# EUROPEAN PATENT SPECIFICATION

(11) **EP 3 285 636 B1**
(45) Date of publication and mention of the grant of the patent: **15.02.2023**
(21) Application number: 16729386.9
(22) Date of filing: 21.04.2016
(51) Int. Cl.: A61B 3/028, A61B 3/12

(54) **PHOROPTER SYSTEM AND METHOD OF USE**
PHOROPTERSYSTEM UND VERFAHREN ZUR VERWENDUNG
SYSTÈME DE PHOROPTÈRE ET PROCÉDÉ D'UTILISATION

(30) Priority: 21.04.2015 US 201562150751 P
(43) Date of publication of application: 28.02.2018
(73) Proprietor: Adaptica S.r.l., 35129 Padova (IT)
(72) Inventor: MENEGHINI, Gianluigi, 35030 Selvazzano Dentro (PD) (IT)
(74) Representative: Gallo, Luca
(86) International application number: PCT/IT2016/000099
(87) International publication number: WO 2016/170554

(56) References cited:
- WO-A1-2011/034638
- US-A- 5 430 508
- US-A1- 2006 106 426
- US-A1- 2008 143 960

## Description

### FIELD OF THE INVENTION

The present invention relates to portable phoropter systems which allow for efficient testing of a patient's visual acuity.

### BACKGROUND OF THE INVENTION

Interactive refractors, typically known as a phoropter, are used for testing the subjective vision of a patient in which a number of lenses are adjusted for the patient to view through. Many of these conventional phoropter systems require the intervention of a skilled optometrist or ophthalmologist and they are also typically large and stationary devices which are difficult to transport.

While there are a number of objective methods for the direct measurement of the aberrations arising in a wavefront emitted from a point of light focused onto the retina of the eye of the subject. The difference between the aberrated wavefront emitted from the eye and a planar undistorted wavefront is measured using a wavefront analysis system. The output of such an instrument is a map of the refractive properties across the eye, which can be used to determine the form and strength of spectacle lenses for correction of the aberration measured. A number of instruments have been previously developed for performing this wavefront analysis.

However, these instruments have had a number of disadvantages. For instance, the illumination source is partly reflected back towards the sensor array and the intensity of this reflected light may be larger than that of the reflection from the patient's retina making measurement of the retinal reflection difficult. Furthermore, the mode of the beam may be degraded in its path through the phoropter elements.

A number of other adjustable lens systems have been developed for altering the optical power range through various methods such as fluid-filled lenses or electro-active lenses. However, such lenses may be limited in their range for testing and correcting higher order visual disorders. Moreover, such systems may be limited in the testing or accounting for the spatial aberrations which may be inherent in the lenses. Accordingly, there exists a need for phoropter systems which are readily transportable and lightweight.

There is also a need for phoropter systems which can also test for the visual acuity of a patient over a wide range and can correct for higher order aberrations.

Document WO-A-2011/034 638 discloses a portable phoropter system combined with a control system, comprising: a support frame configured to be positioned into proximity to at least one eye of a subject; a first housing attached to said support frame, said first housing containing on-board electronics; at least a first lens assembly coupled to said first housing and attached to said support frame such that a proximal opening of said first lens assembly is positioned into proximity to the at least one eye of the subject, said first lens assembly comprising: a spherical power lens having at least a first lens configured to be electrically actuated and controlled by the electronics contained in said housing, said first lens configured to be adjusted in a continuous manner to correct a spherical power; an astigmatic power lens having at least a second lens configured to be electrically actuated and controlled by the electronics contained in said housing, said second lens configured to be adjusted in a continuous manner to correct for astigmatism; an aberrometric lens having at least a third lens configured to be actuated to provide a variable wavefront controlled by the electronics contained in said housing, said third lens configured to be adjusted in a continuous manner, wherein said spherical power lens, said astigmatic power lens and said aberrometric lens are linearly aligned relative to one another, said control system having a phoropter interface configured to receive said phoropter system, a wavefront sensor.

### SUMMARY OF THE INVENTION

A portable phoropter system to be used in combination with a control system as a phoropter assembly is disclosed in claim 1.

The control system may include a control interface through which a user or the patient may interface to input and/or obtain various parameters and can coordinate commands to a connected chart. Once the patient has finished a visual examination using the phoropter system, the phoropter may be positioned upon a phoropter receiving interface on the control interface which may then read and/or download data or parameters related to the measurement of the lenses and aberrations from the phoropter for further processing.

The portable phoropter system comprises a support frame configured to be worn or positioned into proximity to at least one eye of by a subject, at least one lens assembly attached to the support frame such that a proximal opening of the lens assembly is positioned in proximity to at least one eye of the subject. The lens assembly comprises a spherical power lens having at least a first lens which is adjustable to correct a spherical power, an astigmatic power lens having at least a second lens which is adjustable to correct for astigmatism, and an aberrometric lens having at least a third lens which is adjustable to correct for aberrational wavefronts introduced by at least the spherical power lens and astigmatic power lens.

In use, one method for testing the visual acuity of the subject with the portable phoropter system may generally comprise providing the support frame configured to be worn or positioned into proximity to at least one eye of by the subject such that the proximal opening of the at least one lens assembly attached to the support frame is positioned in proximity to at least one eye of the subject, adjusting the spherical power lens of the lens assembly having at least the first lens to correct a spherical power, adjusting the astigmatic power lens having at least the second lens to correct for astigmatism, and adjusting the aberrometric lens having at least the third lens to correct for aberrational wavefronts introduced by at least the spherical power lens and astigmatic power lens.

Another variation of the portable phoropter system may include a system which is positionable upon a platform rather than worn by the patient. For instance, the portable phoropter system may be positioned upon a table in front of the patient without requiring any direct contact with the patient. The phoropter system may have a first phoropter lens assembly, which may be positioned in front of the patient's left eye, and a first housing and a second phoropter lens assembly, which may be positioned in front of the patient's right eye, and a second housing all mounted upon a support frame which may be worn by the patient.

Because both the phoropter system and control system may be portable, the assembly may be utilized, e.g., with a tablet or display, for testing the patient wearing the phoropter system. The table or display may be configured to display a visual for an eye exam (e.g., eye chart) that the patient may view while wearing the phoropter system. Once the examination has been completed, the phoropter system may be positioned upon the control system to download parameters related to the measurements taken by the phoropter system.

The phoropter system may be worn by the patient as they would a pair of glasses. Because the first phoropter lens assembly and the second phoropter lens assembly are automatically positioned respectively in front of the patient's left eye and right eye, the phoropter system allows the patient to view not only the chart for examination purposes but other targets as well or even views outdoors whereas conventional phoropters cannot.

Furthermore, because the first phoropter lens assembly and the second phoropter lens assembly are positioned in front of the patient's eyes at a certain distance from the eye vertex and first lens surface, the power of the lens depends upon this distance and the value of the lenses can be automatically compensated in value because of the continuous regulation of powers whereas conventional phoropters are unable to account for this distance. For instance, the values for sphere and cylinder can be adjusted in a continuous manner so that the resulting values are continuous rather than being stepped in increments of 0.25 diopters which is a limitation of conventional phoropters.

The change of distance between the eyes and glass surface can change those values. For example, in one instance a patient may require a correction of -2 D at 12 mm of this distance. Yet changing the distance between the eyes and glass surface will change the value of the lens that the phoropter indicates to have a -2 D effect. The phoropter system may account for this distance with a continuous variation of lenses adjustment so that an accurate reading may result, e.g., an actual value of 1.89 D. The phoropter system utilizes adaptive optics which can change the optical wavefronts to alter the viewed images and it may do so in a continuous mode rather than being stepped because the phoropter system is able to automatically adjust the optical parameters in its lenses continuously when worn in use by the patient. The phoropter system may incorporate up to three separate lenses for each yet where the lenses are aligned in series. In one variation, the phoropter system may be configured to correct for spherical aberrations and astigmatism to provide conventional data relating to the optical parameters for spherical correction, cylindrical correction, and axis.

The phoropter system is configured to include a wavefront sensor to perform aberrational measurements to correct the spherical lens and astigmatic lens combined into a complete phoropter system. The inclusion of an aberrometric lens may continuously correct for aberrational wavefronts introduced by an astigmatic power lens and a spherical power lens to create an overall flattened wave. Such aberrational wavefronts are typically inherent with spherical lenses due to residuals (e.g., by liquid flow or other lens imperfections). These aberrational wavefronts are generally corrected by tables but the inclusion of an aberrometric lens in combination with an astigmatic power lens and a spherical power lens allows for an optimized solution which provides for a faster and more efficient examination and diagnosis.

Aside from correcting the aberrational wavefronts from the lens assembly, the aberrometric lens may also correct for wavefront errors from the patient's eye(s) as detected by a wavefront sensor. Hence, wavefront correction can reduce all main errors on the wavefront from both the lens assembly as well as from the patient's eyes. Additionally, the phoropter system may also be programmed to perform an in-circuit detection and/or calibration of the wavefront.

Each of the lens assemblies may be coupled to respective first and second housings attached to the frame. The housings may contain a power supply, individual controller, and/or other electronics such as a wireless transmitter and/or receiver, etc. In other variations, the frame may be connected via a wire cable one for each side of the phoropter system, These cables can be then joined into a single cable (e.g., such as headphone wires) which may then be coupled to the control system which may provide a wired connection for data transmission, power (e.g., DC power supply), etc. The aligned lenses may collectively form a channel through which the patient may look through for examination purposes. The aberrometric lens is configured to be actuated to provide a variable wavefront which is configured to be controlled by the on-board electronics contained within the housing. The astigmatic power lens may have lenses aligned in series where the lenses may be actuated to counter-rotate to correct for astigmatism. The spherical power lens as well as the astigmatic power lens are configured to be electrically actuated and controlled by the by the on-board electronics contained within the housing.

As previously described, the aberrometric lens is configured to continuously correct for aberrational wavefronts introduced by the astigmatic power lens and the spherical power lens to create an overall flattened wave. The inclusion of the aberrometric lens in combination with an astigmatic power lens and the spherical power lens allow for an optimized solution. In order to correct for the aberrational wavefronts, the aberrometric lens may locally deform individual regions of the lens through various mechanisms (e.g., liquid, etc.) to create a resultant deformations which can take on any number of complex lens profiles.

The control system is used to obtain measurements from the phoropter system after completion of an eye examination by the patient by obtaining measurement data or parameters with a wavefront sensor. The controller assembly within the control system may include a mounting support having a phoropter interface attached on a first surface where the phoropter interface may define a curved surface or channel for receiving and positioning one of the phoropter lenses so that the lens is aligned with the light emitting opening defined through the phoropter interface and through the mounting support. After the first phoropter lenses has been analyzed, the stage which is mounted upon a runner may be translated to support the phoropter system while the remaining lens is mounted within the phoropter interface and analyzed by the control system. Because the distance between the phoropter lenses is not fixed and may be adjusted (e.g., manually by the operator or patient), the control system has the capability to detect the second lens position automatically analyzing the wavefront.

An illuminator tube may be coupled to the second surface which is opposite to the first surface of the mounting support. The terminal end of the illuminator tube may be optically coupled to a light source, e.g., LED, etc., which may be used to emit a light for transmission through the illuminator tube, mounting support, phoropter interface, and into the phoropter lens. The light which is transmitted through the phoropter lens may be received by the light receiving opening which is positioned in apposition to the light emitting opening. The light receiving opening may be mounted upon a tube support which may then transmit the received light through tube mounted on the lower deck. All operation may be executed in a fully automated manner.

This light may then pass through tube lens and rear light guide. The terminal end of the rear light guide may be attached to a first side of a support which may also have a wavefront sensor guide attached to a second side of the support. The transmitted light is then passed through a direct wavefront sensor analyzing the point spread function or through a pyramid-shaped wavefront sensor which is configured to balance the light onto the complementary metal-oxide-semiconductor (CMOS) or charge-coupled device (CCD) mounted upon the controller board. The wavefront sensor is configured to help automatically detect a position of the lens. The wavefront information detected by the CMOS or CCD when transmitted through the phoropter system may be processed by the control system to provide an accurate measurement of the patient's vision as indicated by the lenses in the phoropter system.

The controller board may be further configured for enabling wired or wireless communication (e.g., infrared, wifi, Bluetooth^{®}, etc.) to a remote computer or server (e.g., cloud connection) to allow for communication and/or transmission of data to remotely located parties such as physicians, technicians, manufacturers, family members, etc.

Prior to use of the phoropter assembly, optical parameters of a patient's visual acuity may be initially input into the phoropter system and/or control system to provide a starting point prior to the patient's examination. With this starting point, the adjustments obtained by the phoropter system may serve as a fine adjustment and/or verification to more quickly correct the patient's vision.

The initial data may be obtained from various sources such as a conventional refractor or from other devices such as auto-refractors. Once such device which may be used in combination with the phoropter assembly may include the 2WIN^{™} (Adaptica Srl, Padova, Italy) refractor which is a binocular handheld refractometer and vision analyzer used to automatically measure refractive errors (e.g., myopia, hyperopia, astigmatism), pupil parameters (e.g., pupil size, pupil distance) and other sight anomalies. Once the initial parameters have been obtained, this data may be transmitted (e.g., wired or wirelessly) directly to the phoropter system and/or controller system to serve as an initial starting point for the patient's vision examination using the phoropter system.

### BRIEF DESCRIPTION OF THE DRAWINGS

Fig. 1A shows a perspective assembly view of a portable phoropter system positioned upon a control system.
Figs. 1B and 1C show alternative perspective views of the phoropter system mounted upon and removed from the control system.
Fig. 1D shows a top view of a variation of the portable phoropter system.
Fig. 1E shows a top view of a variation of the control system.
Figs. 2A and 2B show perspective assembly views of the control system optionally used with a tablet device for patient testing.
Fig. 3 shows a perspective view of a patient wearing the portable phoropter system.
Fig. 4 shows a perspective view of a variation of the portable phoropter system.
Figs. 5A to 5C show respective front, back, and side views of the portable phoropter system.
Fig. 6 shows a cross-sectional side view of the lens assemblies contained within phoropter system.
Figs. 7A and 7B show various cross-sectional views of the lens assemblies within the phoropter system.
Figs. 8A and 8B show perspective views of one of the lens assembly housing and lens assembly of the phoropter system.
Fig. 9 shows an exploded assembly view of the individual aberrometric lens, astigmatic power lens, and spherical power lens assemblies which may be incorporated into the phoropter system.
Figs. 10A to 10C show various perspective views of the astigmatic power lens and some of its components.
Fig. 11 shows an example of a wave-front map of the aberrometric lens when actuated.
Figs. 12A to 12C show examples of plots illustrating wave-front residuals, wave-front error vs. focal power, and current vs. focal power.
Fig. 13 shows an example of the spatial profile of the astigmatic power lens when actuated.
Figs. 14A and 14B show various perspective views of the control system.
Figs. 15A and 15B show various perspective views of the optical and electronics assemblies within the control system.
Figs. 16A and 16B show various perspective views of the optical assembly within the control system.
Figs. 17A and 17B show side and partial cross-sectional detail views of the optical assembly.
Fig. 18 shows a perspective view of the control board assembly.
Fig. 19 shows an example of an apparatus for detecting ocular defects which may be used in combination with the control system.

### DETAILED DESCRIPTION OF THE INVENTION

A portable phoropter system which can be worn by a patient is described where the phoropter system is able to be implemented on glasses frames, a helmet, or other wearable platforms. While the portable phoropter system can be optionally provided on a stationary platform, the portability of the phoropter allows for the patient to freely look around at different objects as in normal use to provide for more accurate data. The portable phoropter can also easily be used in a table stand or standard stand.

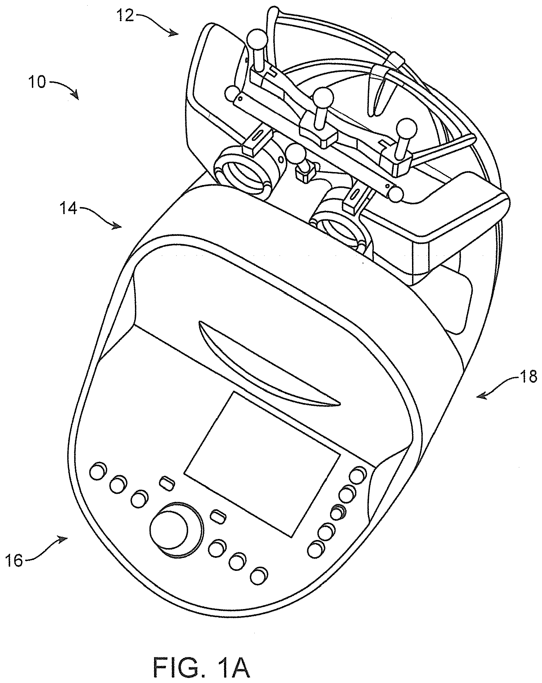

The portable phoropter system 12, as shown in the assembly view of Fig. 1A, may be used in combination with a control system 14 as a phoropter assembly 10. The control system 14 may include a control interface 16 through which a user or the patient may interface to input and/or obtain various parameters and can coordinate commands to a connected chart. Once the patient has finished a visual examination and the corresponding corrections to their vision have been obtained using the phoropter system 12, the phoropter 12 may be positioned upon a phoropter receiving interface 18 on the control interface 16, as described in further detail herein, which may then read and/or download the data or parameters related to measurements of the lenses and aberrations from the phoropter 12 for further processing, e.g., manufacturing eyeglasses, further assessment, etc. The final output of the system may include, e.g., the correction lenses prescriptions confirmation of the subjective exam.

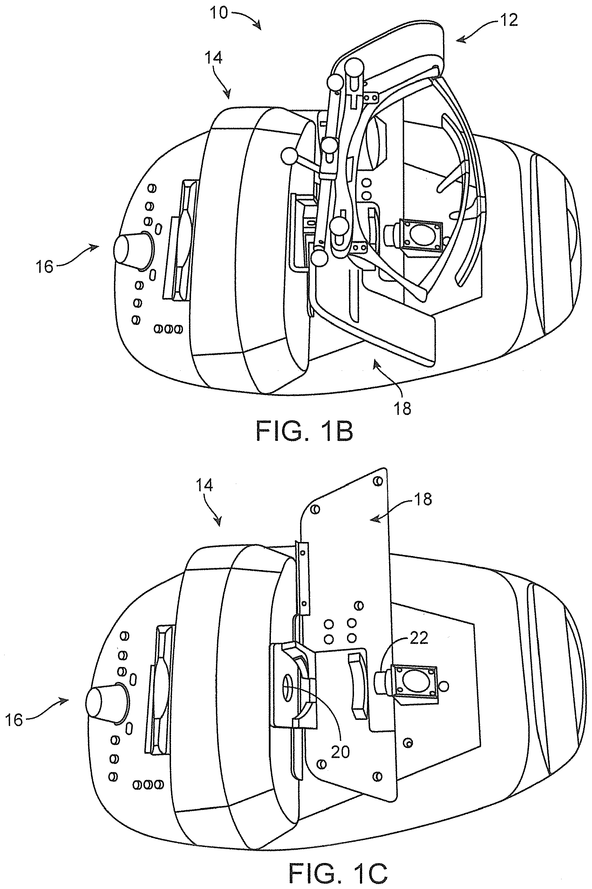

Fig. 1B shows the phoropter assembly 10 with the portable phoropter system 12 positioned upon the control system 14 for obtaining the measurements from the phoropter system 12. Fig. 1C shows the control system 14 with the phoropter system 12 removed to illustrate the phoropter receiving interface 18 on the control interface 16 and the light emitting opening 20 through which light is emitted into the phoropter system 12 and the light receiving opening 22 through which the light transmitted through the phoropter 12 is received for processing by the control system 14.

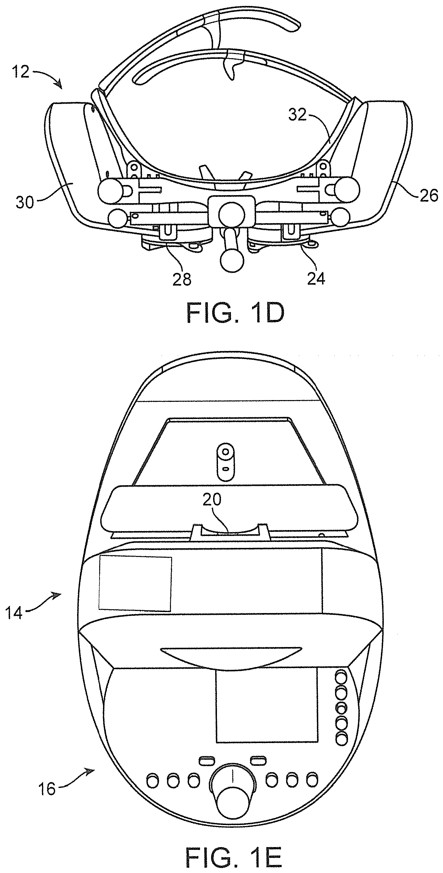

Fig. 1D shows a top view of the phoropter system 12 configured similarly to an eyeglass frame in this variation to be worn by the patient in a similar manner. The phoropter system 12 may have a first phoropter lens assembly 24, which may be positioned in front of the patient's left eye, and a first housing 26 and a second phoropter lens assembly 28, which may be positioned in front of the patient's right eye, and a second housing 30 all mounted upon a support frame 32 which may be worn by the patient.

Fig. 1E shows a top view of the control system 14 illustrating the user interface 16.

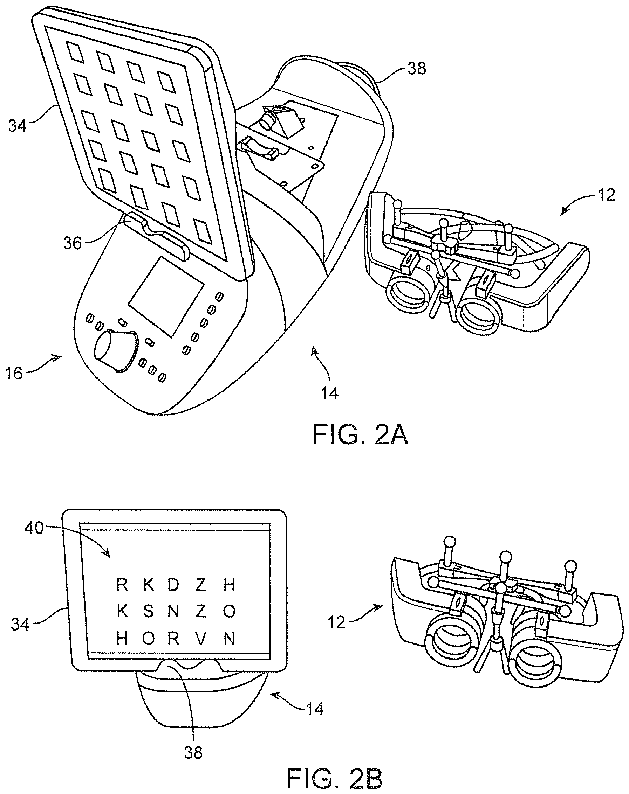

Because both the phoropter system 12 and control system 14 may be portable, the assembly 10 may be utilized, e.g., with a tablet or display 34, optionally positioned upon a first mount 36 or second mount 38 on the control system 14 for testing the patient wearing the phoropter system 12, as shown in the perspective assembly views of Figs. 2A and 2B. The table or display 34 may be configured to display a visual 40 for an eye exam (e.g., eye chart) that the patient may view while wearing the phoropter system 12. Alternatively, a standard eye chart (e.g., placed upon a wall) may also be used. Once the examination has been completed, the phoropter system 12 may be positioned upon the control system 14 to download parameters related to the measurements taken by the phoropter system 12, as described in further detail herein.

Another variation of the portable phoropter system may include a system which is positionable upon a platform rather than worn by the patient. For instance, the portable phoropter system may be positioned upon a table in front of the patient without requiring any direct contact with the patient.

### PHOROPTER SYSTEM

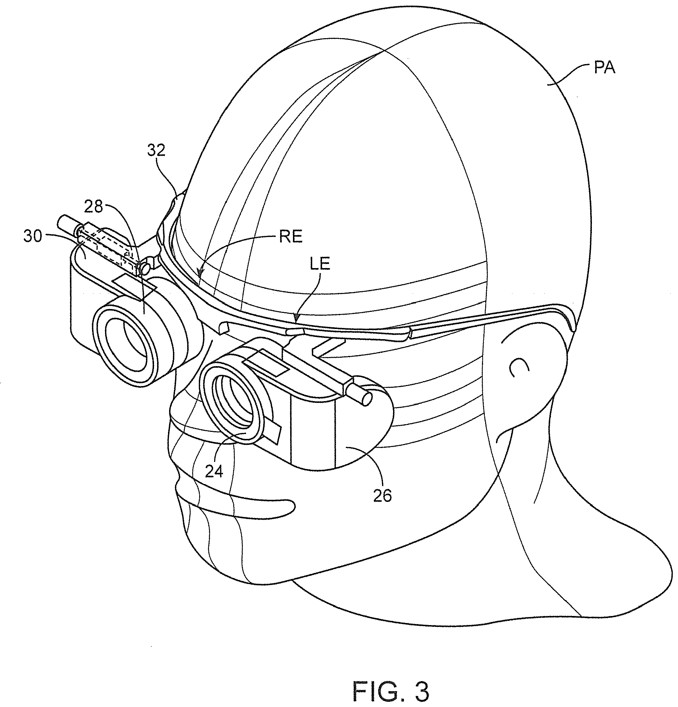

As discussed above, the phoropter system 12 may be worn by the patient PA as they would a pair of glasses, as shown in the perspective view of Fig. 3. Because the first phoropter lens assembly 24 and the second phoropter lens assembly 28 are automatically positioned respectively in front of the patient's left eye LE and right eye RE, the phoropter system 12 allows the patient to view not only the chart for examination purposes but other targets as well or even views outdoors whereas conventional phoropters cannot. Although two lens assemblies are shown 24, 28 in the phoropter system 12, other variations may have a single lens assembly configured to be positioned in proximity to at least one eye of the patient. In such a variation, the single lens assembly may be used to examine at least one or both eyes of the patient.

The phoropter system 12 utilizes adaptive optics which can change the optical wavefronts to alter the viewed images and it does so in a continuous mode rather than being stepped because the phoropter system 12 is able to automatically adjust the optical parameters in its lenses continuously when worn in use by the patient. The phoropter system 12 may incorporate up to three separate lenses for each yet where the lenses are aligned in series. In one variation, the phoropter system 12 may be configured to correct for spherical aberrations and astigmatism to provide conventional data relating to the optical parameters for spherical correction, cylindrical correction, and axis.

Furthermore, because the first phoropter lens assembly and the second phoropter lens assembly are positioned in front of the patient's eyes at a certain distance from the eye vertex and first lens surface, the power of the lens depends upon this distance and the value of the lenses can be automatically compensated in value because of the continuous regulation of powers whereas conventional phoropters are unable to account for this distance. For instance, the values for sphere and cylinder can be adjusted in a continuous manner so that the resulting values are continuous rather than being stepped in increments of 0.25 diopters which is a limitation of conventional phoropters.

The change of distance between the eyes and glass surface can change those values. For example, in one instance a patient may require a correction of -2 D at 12 mm of this distance. Yet changing the distance between the eyes and glass surface will change the value of the lens that the phoropter indicates to have a -2 D effect. The phoropter system may account for this distance with a continuous variation of lenses adjustment so that an accurate reading may result, e.g., an actual value of 1.89 D.

In another variation, the phoropter system 12 may also be configured to optionally include aberrational measurements to correct the spherical lens and astigmatic lens combined into a complete phoropter system 12. The inclusion of an aberrometric lens may continuously correct for aberrational wavefronts introduced by an astigmatic power lens and a spherical power lens to create an overall flattened wave. Such aberrational wavefronts are typically inherent with spherical lenses due to residuals (e.g., by liquid flow or other lens imperfections). These aberrational wavefronts are generally corrected by tables but the inclusion of an aberrometric lens in combination with an astigmatic power lens and a spherical power lens allows for an optimized solution which provides for a faster and more efficient examination and diagnosis.

Aside from correcting the aberrational wavefronts from the lens assembly, the aberrometric lens may also correct for wavefront errors from the patient's eye(s) as detected by a wavefront sensor. Hence, wavefront correction can reduce all main errors on the wavefront from both the lens assembly as well as from the patient's eyes. Additionally, the phoropter system may also be programmed to perform an in-circuit detection and/or calibration of the wavefront.

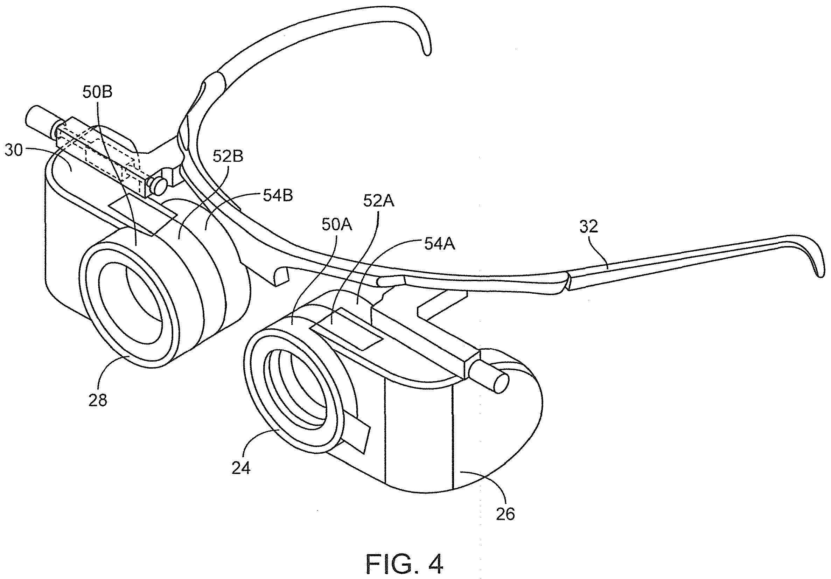

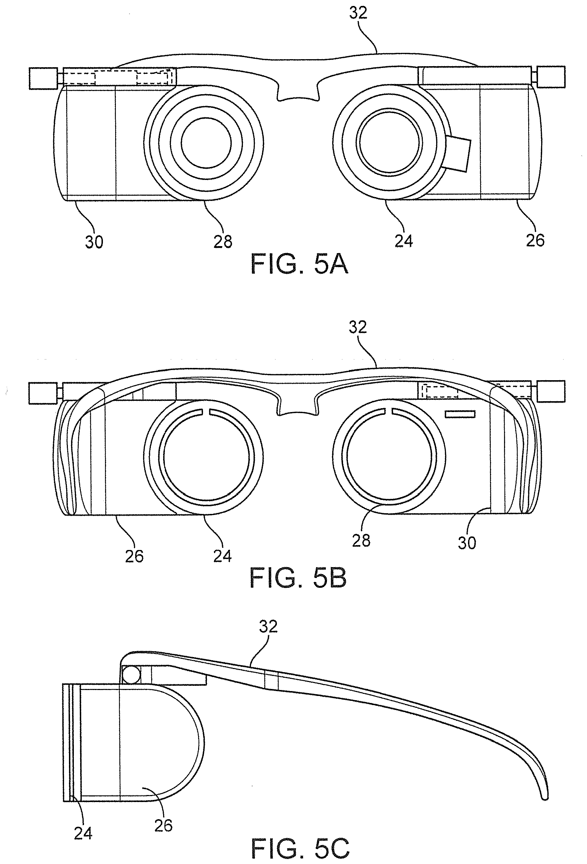

Fig. 4 shows a perspective view of a variation of the phoropter system 12 having respective first and second aberrometric lens 50A, 50B aligned distally with respective first and second astigmatic power lens 52A, 52B and distally with respective first and second spherical power lens 54A, 54B. Each of the lens assemblies 24, 28 may be coupled to respective first and second housings 26, 30 attached to the frame 32. The housings 26, 30 may contain a power supply, individual controller, and/or other electronics such as a wireless transmitter and/or receiver, etc. In other variations, the frame 32 may be connected via a wire cable one for each side of the phoropter system 12. These cables can be then joined into a single cable (e.g., such as headphone wires) which may then be coupled to the control system 14 which may provide a wired connection for data transmission, power (e.g., DC power supply), etc. Figs. 5A to 5C show respective front, back, and side views of the phoropter system 12 of Fig. 4 illustrating how the housings 26, 30 may be optionally contoured to fit around the patient's eyes and head while maintaining the lens assemblies 24, 28 positioned in front of the patient's eyes.

While the variation illustrates the aberrometric lens 50A, 50B positioned distal to the astigmatic power lens 52A, 52B which is also distal to the spherical power lens 54A, 54B, other variations of the phoropter system 12 may have the lenses aligned in different combinations and the phoropter system 12 is not limited to the particular order of alignment of the lenses.

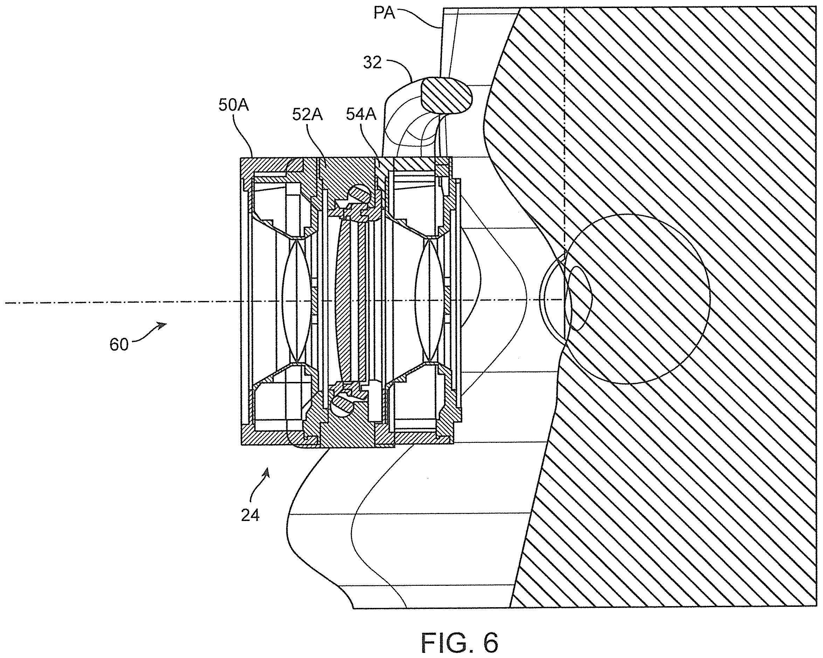

Fig. 6 shows a cross-sectional side view of the phoropter system 12 situated in front of the patient's eyes. The variation shown illustrates how the aligned lenses 50A, 52A, 54A may collectively form a channel 60 through which the patient PA may look through for examination purposes. The field of view, shown in this example as 32°, may range depending upon the diameter of the lenses and the distance between the phoropter system 12 and the patient's eyes. The aberrometric lens 50A, 50B may be actuated to provide a variable wavefront which may be controlled by the on-board electronics contained within the housing 26, 30.

The variation shown may have a diameter of, e.g., 20 mm, with a static aberration of less than, e.g., 0.8 µm RMS. The maximum stroke may be, e.g., 5 µm, although this may be varied. Also, the aberrometric lens 50A, 50B may contain a volume of liquid, e.g., 40 µL, which may be used to actuate the lens.

The astigmatic power lens 52A may also be seen where the lens may have lenses aligned in series where the lenses may be actuated to counter-rotate to correct for astigmatism. The astigmatic power lens 52A, 52B may have a diameter of, e.g., 20 mm, with a dioptric range of, e.g., ± 10 Diopters and a static aberration of less than, e.g., 1 µm RMS. The variable axis may range from, e.g., 0° to 360°, and the lens may have an accuracy of, e.g., ± 0.125 Diopters.

The spherical power lens 54A, 54B may also have a diameter of, e.g., 16 mm, and have a dioptric range of, e.g., ± 10 Diopters, with a static aberration of less than, e.g., 0.3 µm RMS, and an accuracy of, e.g., ± 0.125 Diopters. The spherical power lens 54A, 54B as well as the astigmatic power lens 52A, 52B are configured to be electrically actuated and controlled by the by the on-board electronics contained within the housing 26, 30. The values presented for each of the lenses are intended to be illustrative and not limiting. Accordingly, the values may be varied depending upon the applications and lenses utilized.

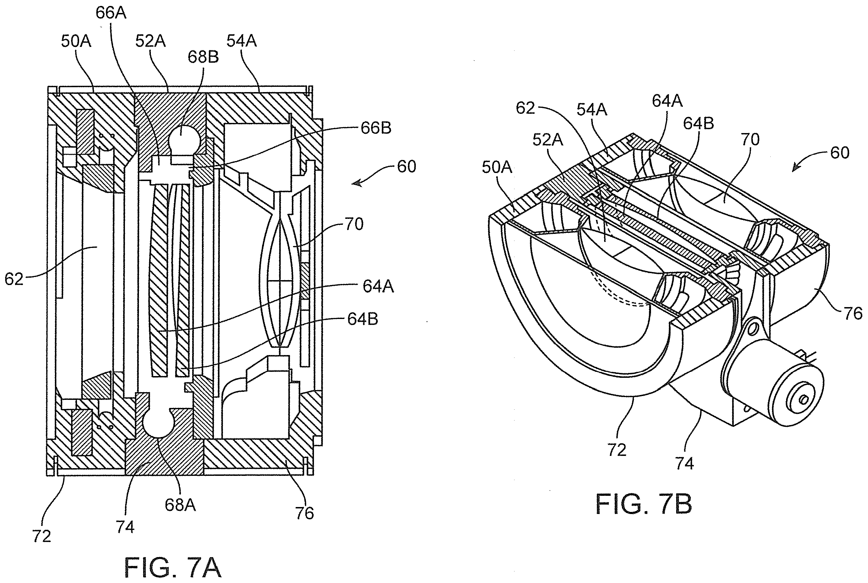

Figs. 7A and 7B show further of cross-sectional side views to illustrate examples of how the various lenses may be positioned relative to one another. The aberrometric lens 50A may be seen having an actuatable lens 62 positioned within an aberrometric lens housing 72 and astigmatic power lens 52A may also be seen having an astigmatic power lens housing 74 with a counter-rotating first lens 64A supported within a first lens frame 66A and a second lens 64B supported within an adjacent second lens frame 66B. The first lens frame 66A may be engaged to a first worm gear 68A while the second lens frame 66B may be similarly engaged to a second worm gear 68B positioned along the opposite side of the lens assembly. The spherical power lens 54A may also be seen having spherical lens 70 positioned within a spherical power lens housing 76.

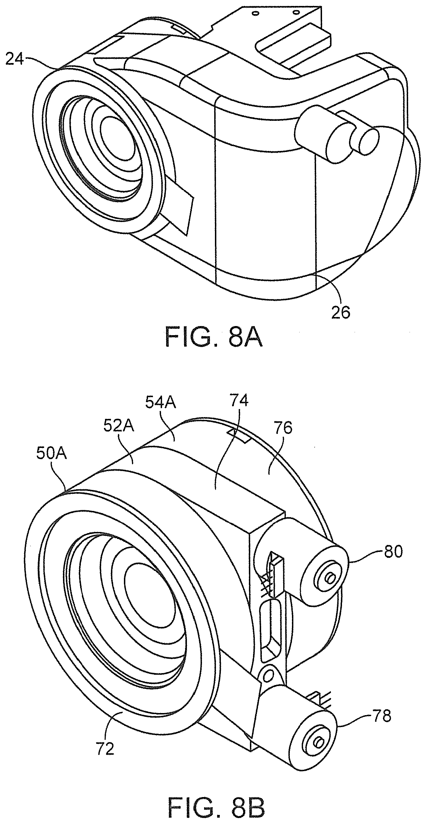

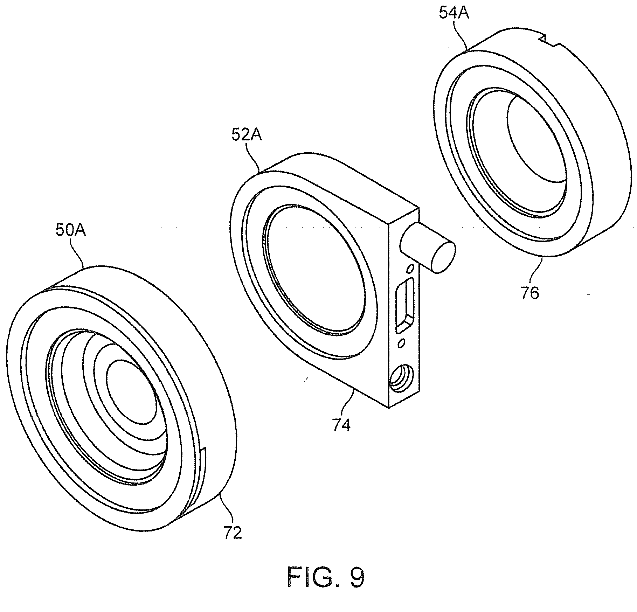

Fig. 8A and 8B show additional perspective views of the first lens assembly 24 and corresponding housing 26 as well as the lens assembly 24 with each of the respective lens housings 72, 74, 76. The housing 26 may also integrate a first actuator 78 and a second actuator 80 coupled to the respective first worm gear 68A and second worm gear 68B for actuating the counter-rotating lenses 64A, 64B. Fig. 9 shows an exploded perspective assembly view of the individual lenses 50A, 52A, 54A separated from one another. When aligned in the phoropter system 12, each of the housings 72, 74, 76 may be coupled to an adjacent lens housing such that an individual lens may be removed and replaced.

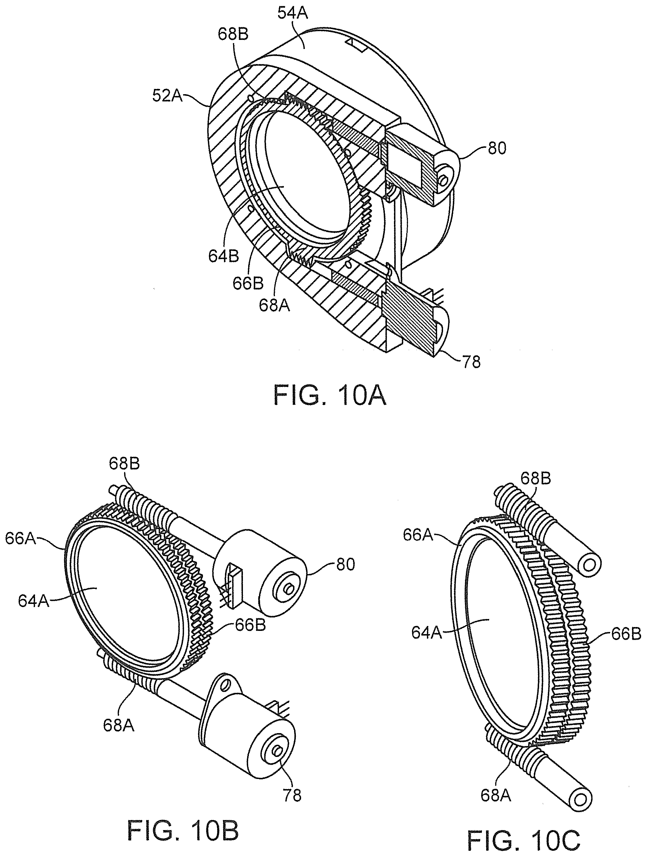

Figs. 10A to 10C show perspective detail views of the assembly of the astigmatic power lens 52A in detail to illustrate how the first and second lens frames 66A, 66B may be configured as geared structures for engagement with the respective first and second worm gears 68A, 68B.

As the respective first and second actuators 78, 80 are activated to rotate worm gears 68A, 68B about their longitudinal axes, the engagement with lens frames 66A, 66B may cause the lenses 64A, 64B to counter-rotate while under the control of the on-board electronics and controller.

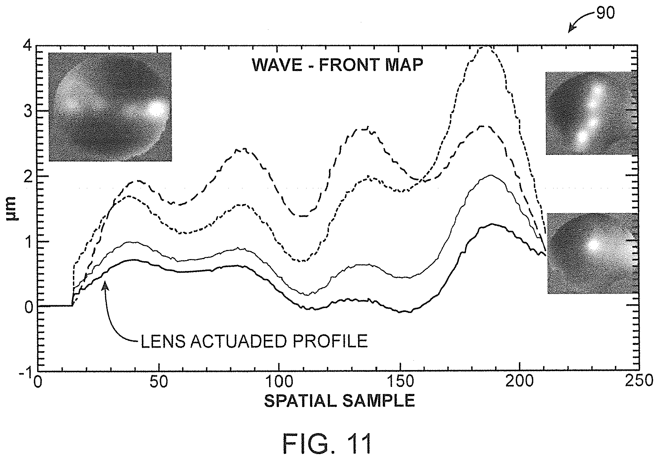

As previously described, the aberrometric lens 50A, 50B may continuously correct for aberrational wavefronts introduced by the astigmatic power lens 52A, 52B and the spherical power lens 54A, 54B to create an overall flattened wave. The inclusion of the aberrometric lens 50A, 50B in combination with an astigmatic power lens 52A, 52B and the spherical power lens 54A, 54B allow for an optimized solution. In order to correct for the aberrational wavefronts, the aberrometric lens 50A, 50B may locally deform individual regions of the lens through various mechanisms (e.g., liquid, etc.) to create a resultant deformations which can take on any number of complex lens profiles, as shown in Fig. 11. The wavefront map 90 illustrates examples of various lens profiles which may be formed by the aberrometric lens 50A, 50B as automatically controlled by the on-board processor within the housing 26, 30.

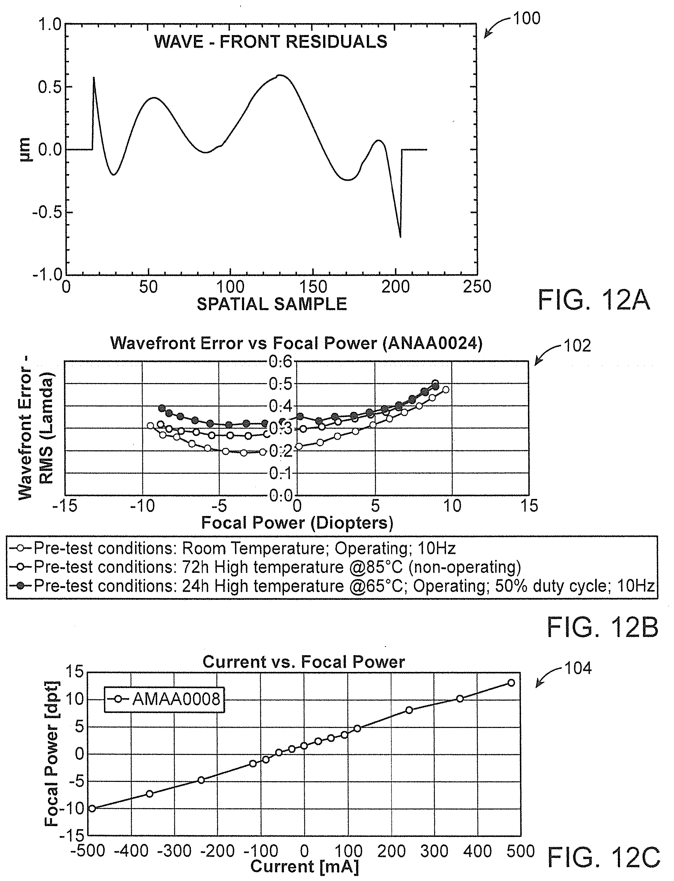

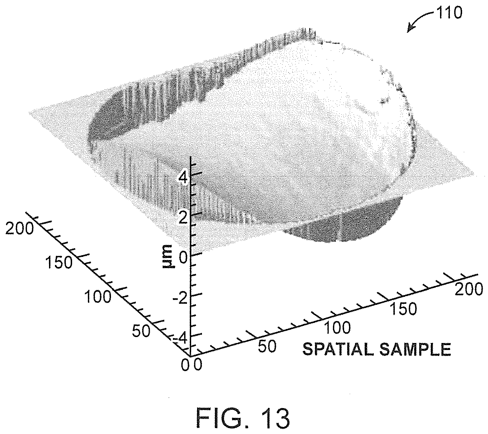

The spherical power lens 54A, 54B may introduce residual wavefront aberrations, as illustrated by the plot of wavefront residuals 100 of a spatial sample in Fig. 12A. Fig. 12B shows a plot of wavefront error vs. focal power 102 to illustrate the RMS wavefront error relative to the focal power (Diopters) of the actuated lens 54A, 54B at various pre-test conditions (e.g., room temperature, 65° C, 85° C). Fig. 12C shows a plot of current (mA applied) vs. focal power (Diopters) 104 of the lens 54A, 54B when actuated. Nonetheless, the overall static aberration of the spherical power lens 54A, 54B is less then, e.g., 0.3 µm RMS. The RMS of the wavefront residual after propagation through the spherical power lens 54A, 54B may nonetheless be corrected by the aberrometric lens 50A, 50B. Like the spherical power lens 54A, 54B, the astigmatic power lens 52A, 52B (used for astigmatic correction) may also introduce static aberrations into the wavefront of less than, e.g., 1 µm RMS. An example of an astigmatic profile 110 which may be corrected by the astigmatic power lens 52A, 52B is shown in Fig. 13. The static aberrations introduced by the astigmatic power lens 52A, 52B may also be corrected by the aberrometric lens 50A, 50B.

### CONTROL SYSTEM

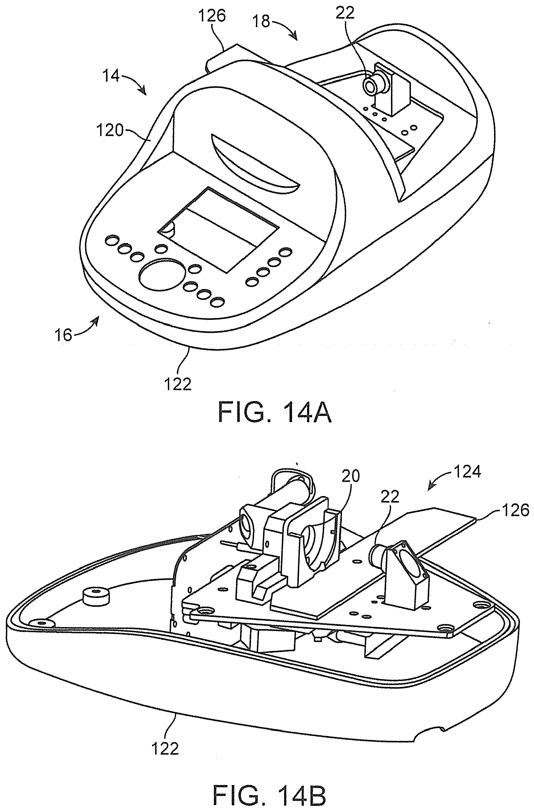

Turning now to the control system 14 used in combination with the phoropter system 12, the control system 14 may be used to obtain measurements from the phoropter system 12 after completion of an eye examination by the patient by obtaining measurement data or parameters with a wavefront sensor. Figs. 14A and 14B show perspective views of the control system 14 and the upper housing 120 and lower housing 122 which may enclose a controller assembly 124. Fig. 14B shows the upper housing 120 removed for clarity. The translating stage 126 upon which the phoropter system 12 is positionable may also be seen.

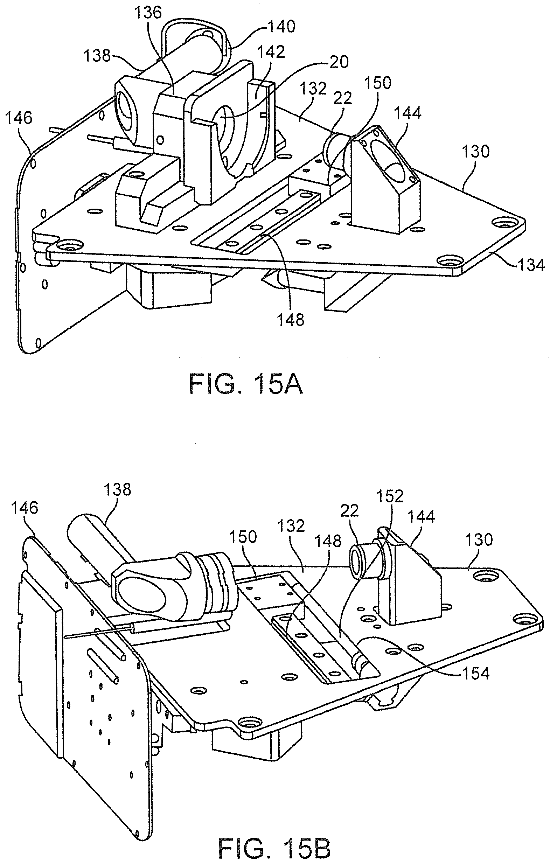

The controller assembly 124 is further shown in the perspective views of Figs. 15A and 15B to illustrate the upper deck 132 of the platform 130 upon which several of the components are mounted or positioned. Additional components are further mounted on the lower deck 134 of the platform 130 and are described in further detail herein. A mounting support 136 may have a phoropter interface 142 attached on a first surface where the phoropter interface 142 may define a curved surface or channel for receiving and positioning one of the phoropter lenses 24, 28 so that the lens is aligned with the light emitting opening 20 defined through the phoropter interface 142 and through the mounting support 136. After the first phoropter lenses 24 has been analyzed, the stage 126 which is mounted upon runner 150 may be translated within the opening 154 defined through the platform 130 and along guide 148 via shaft 152 to support the phoropter system 12 while the remaining lens 28 is mounted within the phoropter interface 142 and analyzed by the control system 14. Because the distance between the phoropter lenses is not fixed and may be adjusted (e.g., manually by the operator or patient), the control system 14 has the capability to detect the second lens position automatically analyzing the wavefront.

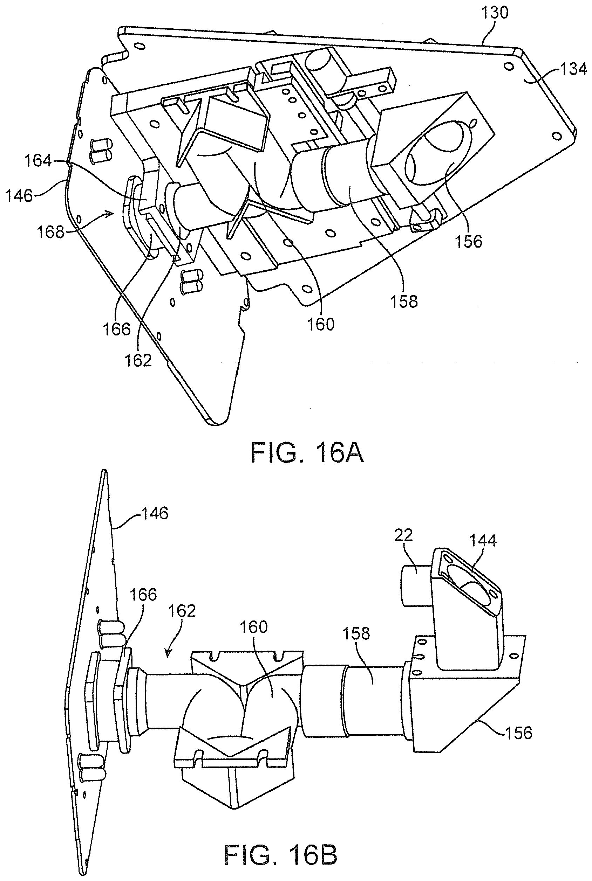

An illuminator tube 138 may be coupled to the second surface which is opposite to the first surface of the mounting support 136. The terminal end of the illuminator tube 138 may be optically coupled to a light source 140, e.g., LED, etc., which may be used to emit a light for transmission through the illuminator tube 138, mounting support 136, phoropter interface 142, and into the phoropter lens 24 or 28. The light which is transmitted through the phoropter lens 24 or 28 may be received by the light receiving opening 22 which is positioned in apposition to the light emitting opening 20. The light receiving opening 22 may be mounted upon a tube support 144 which may then transmit the received light through tube 156 mounted on the lower deck 134, as shown in the perspective view of Fig. 16A. All operation may be executed in a fully automated manner.

This light may then pass through tube lens 158 and rear light guide 160, as shown in the perspective view of Fig. 16B which illustrates the assembly with the platform 130 removed for clarity. The terminal end of the rear light guide 160 may be attached to a first side of a support 164 which may also have a wavefront sensor guide 166 attached to a second side of the support 164. The transmitted light is then passed through a direct wavefront sensor analyzing the point spread function or through a pyramid-shaped wavefront sensor 162 which is configured to balance the light onto the complementary metal-oxide-semiconductor (CMOS) or charge-coupled device (CCD) 168 mounted upon the controller board 146. The wavefront sensor 162 may also be configured to help automatically detect a position of the lens 24 or 28. Generally, any number of various wavefront sensors may be utilized (e.g., Shack-Hartmann wavefront sensor) as wavefront sensor 162 which is not limited to any particular type of sensor. The wavefront information detected by the CMOS or CCD 168 when transmitted through the phoropter system 12 may be processed by the control system 14 to provide an accurate measurement of the patient's vision as indicated by the lenses in the phoropter system 12.

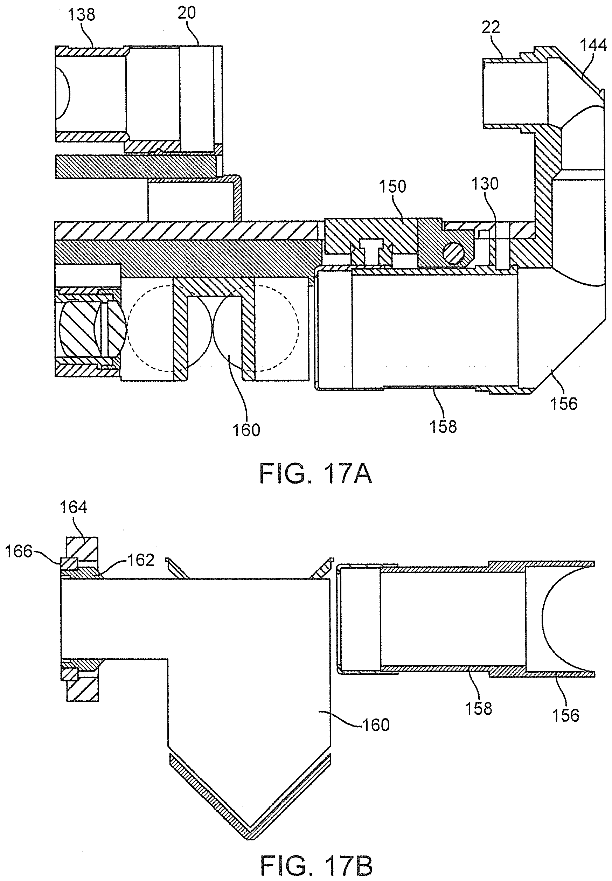

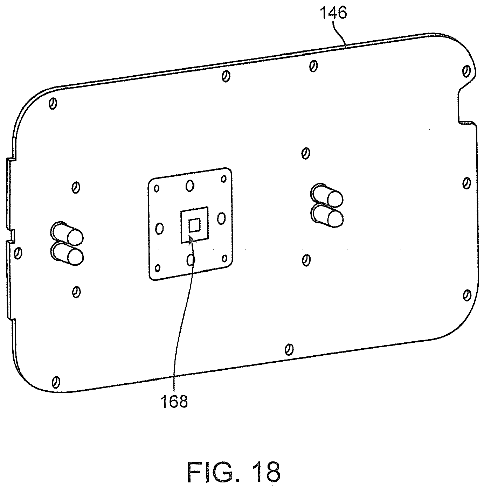

Figs. 17A and 17B show partial cross-sectional side and top views of the illumination pathway to illustrate how the emitted light may be transmitted through the illuminator tube 138 and out of the light emitting opening 20 for transmission through the phoropter lens and then into the light receiving opening 22. The light is then transmitted through the tube support 144 and tube 156 mounted on the lower deck 134, and then through tube lens 158 and rear light guide 160 where the light is then transmitted through the wavefront sensor 162 and ultimately on to the CMOS or CCD 168 mounted on the controller board 146, shown in the perspective view of Fig. 18, for detection and processing by a processor mounted on the controller board 146.

The controller board 146 may be further configured for enabling wired or wireless communication (e.g., infrared, wifi, Bluetooth^{®}, etc.) to a remote computer or server (e.g., cloud connection) to allow for communication and/or transmission of data to remotely located parties such as physicians, technicians, manufacturers, family members, etc.

Prior to use of the phoropter assembly 10, optical parameters of a patient's visual acuity may be initially input into the phoropter system 12 and/or control system 14 to provide a starting point prior to the patient's examination. With this starting point, the adjustments obtained by the phoropter system 12 may serve as a fine adjustment and/or verification to more quickly correct the patient's vision.

The initial data may be obtained from various sources such as a conventional refractor or from other devices such as auto-refractors. Once such device which may be used in combination with the phoropter assembly 10 may include the 2WIN^{™} (Adaptica Sri, Padova, Italy) refractor which is a binocular handheld refractometer and vision analyzer used to automatically measure refractive errors (e.g., myopia, hyperopia, astigmatism), pupil parameters (e.g., pupil size, pupil distance) and other sight anomalies.

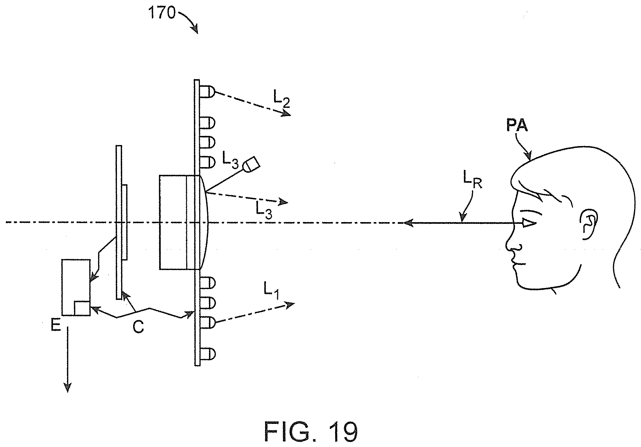

Such an ocular defect detection apparatus 170 is shown illustratively in Fig. 19 for initially obtaining parameters relating to the vision of the patient PA. Once the initial parameters have been obtained, this data may be transmitted (e.g., wired or wirelessly) directly to the phoropter system 12 and/or controller system 14 to serve as an initial starting point for the patient's vision examination using the phoropter system 12.

The applications of the devices and methods discussed above are not limited to the examples and variations described herein but may include any number of further variations and combinations. Modification of the above-described assemblies and methods for carrying out the invention, combinations between different variations as practicable, and variations of aspects of the invention that are obvious to those of skill in the art are intended to be within the scope of the claims.

## Claims

1. A portable phoropter assembly (10), comprising a portable phoropter system (12) combined with a control system (14);
said portable phoropter system (12) comprising:
- a support frame (32) configured to be positioned into proximity to at least one eye of a subject;
- a first housing (26) attached to said support frame (32), said first housing (26) containing onboard electronics;
- at least a first lens assembly (24) coupled to said first housing (26) and attached to said support frame (32) such that a proximal opening of said first lens assembly (24) is positioned into proximity to the at least one eye of the subject, said first lens assembly (24) comprising:
- a spherical power lens (54A) having at least a first lens configured to be electrically actuated and controlled by the electronics contained in said first housing (26), said first lens being adjustable in a continuous manner to correct a spherical power;
- an astigmatic power lens (52A) having at least a second lens configured to be electrically actuated and controlled by the electronics contained in said first housing (26), said second lens being adjustable in a continuous manner to correct for astigmatism;
- an aberrometric lens (50A) having at least a third lens that is configured to be actuated to provide a variable wavefront controlled by the electronics contained in said first housing (26), said third lens being automatically adjustable in a continuous manner to correct for aberrational wavefronts introduced by at least the spherical power lens (54A) and astigmatic power lens (52A),
wherein said spherical power lens (54A), said astigmatic power lens (52A) and said aberrometric lens (50A) are linearly aligned relative to one another;
said control system (14) having a phoropter interface (18) configured to receive said phoropter system (12) and comprising a light emitter (20) configured to transmit light through the first lens assembly (24), said phoropter system (12) being configured to receive the
light transmitted through said first lens assembly (24);
said control system (14) comprising a CMOS or CCD (168) configured to detect the light transmitted through said first lens assembly (24);
said control system (14) comprising a light emitting opening (20) through which light is emitted into said phoropter system (12) positioned upon said control system (14), and a light receiving opening (22) through which the light transmitted through said phoropter system (12) is received for processing by said control system (14), for obtaining measurements from said phoropter system (12);
wherein said phoropter system (12) includes a wavefront sensor to perform aberrational measurements to correct said spherical power lens (54A) and said astigmatic power lens (52A) combined into said phoropter system.

2. The portable phoropter assembly (10), according to claim 1, **characterised in that** said support frame (32) is configured to be worn by a subject as an eyeglass frame.

3. The portable phoropter assembly (10), according to claim 1, **characterised in that** it comprises a second lens assembly (28) positioned adjacent to said first lens assembly (24) upon said support frame (32).

4. The portable phoropter assembly (10), according to claim 1, **characterised in that** said spherical power lens (54A) has a static aberration of less than 0.3 µm RMS.

5. The portable phoropter assembly (10), according to claim 1, **characterised in that** said astigmatic power lens (52A) has a static aberration of less than 1 µm RMS.

6. The portable phoropter assembly (10), according to claim 1, **characterised in that** said aberrometric lens (50A) has a static aberration of less than 0.8 µm RMS.

7. The portable phoropter assembly (10), according to one of the previous claims, **characterised in that** said control system (14) further comprises a controller board (146) configured to control the control system (14).

8. The portable phoropter assembly (10), according to one of the previous claims, **characterised in that** said phoropter system (12) and said control system (14) are in wireless communication with one another.

9. The portable phoropter assembly (10), according to one of the previous claims, **characterised in that** said control system (14) is in wireless communication with a server remote from the control system.

## Patentansprüche

1. Tragbares Phoroptersystem (10), umfassend ein tragbares Phoroptersystem (12) kombiniert mit einem Steuerungssystem (14);
wobei das genannte tragbare Phoroptersystem (12) Folgendes umfasst:
- ein Traggestell (32), das darauf ausgelegt ist, in der Nähe mindestens eines Auges einer Person positioniert zu werden;
- ein erstes Gehäuse (26), das an dem genannten Traggestell (32) angebracht ist, wobei das genannte erste Gehäuse (26) Bordelektronik umfasst;
- mindestens eine erste Linsenbaugruppe (24), die mit dem genannten ersten Gehäuse (26) gekuppelt und an dem genannten ersten Traggestell (32) so befestigt ist, dass eine proximale Öffnung der genannten ersten Linsenbaugruppe (24) in der Nähe des mindestens einen Auges der Person positioniert ist, wobei die genannte Linsenbaugruppe (24) Folgendes umfasst:
- ein sphärisches Sehstärkenglas (54A) mit mindestens einer ersten Linse, das darauf ausgelegt ist, von der in dem genannten ersten Gehäuse (26) enthaltenen Elektronik elektrisch betätigt und gesteuert zu werden, wobei die genannte erste Linse auf kontinuierliche Weise verstellt werden kann, um eine sphärische Sehstärke zu korrigieren;
- ein astigmatisches Sehstärkenglas (52A) mit mindestens einer zweiten Linse, das darauf ausgelegt ist, von der in dem genannten ersten Gehäuse (26) enthaltenen Elektronik elektrisch betätigt und gesteuert zu werden, wobei die genannte zweite Linse auf kontinuierliche Weise verstellt werden kann, um einen Astigmatismus zu korrigieren;
- ein aberrometrisches Glas (50A) mit mindestens einer dritten Linse, das darauf ausgelegt ist, betätigt zu werden und so eine von der in dem genannten ersten Gehäuse (26) enthaltenen Elektronik gesteuerte variable Wellenfront bereitzustellen, wobei die genannte dritte Linse automatisch auf kontinuierliche Weise verstellbar ist, um aberrationale Wellenformen zu korrigieren, die von mindestens dem sphärischen Sehstärkenglas (54A) und dem astigmatischen Sehstärkenglas (52A) präsentiert werden,
wobei das genannte sphärische Sehstärkenglas (54A), das genannte astigmatische Sehstärkenglas (52A) und das genannte aberrometrische Glas (50A) im Verhältnis zueinander linear ausgerichtet sind;
wobei das genannte Steuerungssystem (14) eine Phoropterschnittstelle (18) aufweist, die darauf ausgelegt ist, das genannte Phoroptersystem (12) aufzunehmen und einen Lichtemitter (20) umfasst, der darauf ausgelegt ist, über die erste Linsenbaugruppe (24) Licht abzugeben, wobei das genannte Phoroptersystem (12) darauf ausgelegt ist, das über die genannte erste Linsenbaugruppe (24) übertragene Licht zu empfangen; wobei das genannte Steuerungssystem (14) einen CMOS oder CCD (168) umfasst, der darauf ausgelegt ist, das über die genannte erste Linsenbaugruppe (24) übertragene Licht zu erfassen;
wobei das genannte Steuerungssystem (14) eine Licht emittierende Öffnung (20) umfasst, durch die Licht in das auf dem genannten Steuerungssystem (14) positionierte genannte Phoroptersystem (12) emittiert wird, und eine Licht empfangende Öffnung (22), durch die das über das genannte Phoroptersystem (12) übertragene Licht zur Verarbeitung durch das genannte Steuerungssystem (14) empfangen wird, um Messungen von dem genannten Phoroptersystem (12) zu erhalten;
wobei das genannte Phoroptersystem (12) einen Wellenfrontsensor zur Durchführung von Aberrationsmessungen umfasst, um das genannte sphärische Sehstärkenglas (54A) und das genannte astigmatische Sehstärkenglas (52A) in dem genannten Phoroptersytem kombiniert zu korrigieren.

2. Tragbares Phoroptersystem (10) nach Anspruch 1, **dadurch gekennzeichnet, dass** das genannte Traggestell (32) darauf ausgelegt ist, von einer Person als Brillengestell getragen zu werden.

3. Tragbares Phoroptersystem (10) nach Anspruch 1, **dadurch gekennzeichnet, dass** es eine an die genannte erste Linsenbaugruppe (24) angrenzend positionierte zweite Linsenbaugruppe (28) auf dem genannten Traggestell (32) umfasst.

4. Tragbares Phoroptersystem (10) nach Anspruch 1, **dadurch gekennzeichnet, dass** das genannte sphärische Sehstärkenglas (54A) eine statische Aberration von weniger als 0,3 µm RMS aufweist.

5. Tragbares Phoroptersystem (10) nach Anspruch 1, **dadurch gekennzeichnet, dass** das genannte astigmatische Sehstärkenglas (52A) eine statische Aberration von weniger als 1 µm RMS aufweist.

6. Tragbares Phoroptersystem (10) nach Anspruch 1, **dadurch gekennzeichnet, dass** das genannte aberrometrische Glas (50A) eine statische Aberration von weniger als 0,8 µm RMS aufweist.

7. Tragbares Phoroptersystem (10) nach einem der vorangegangenen Ansprüche, **dadurch gekennzeichnet, dass** das genannte Steuerungssystem (14) außerdem eine Controller-Platine (146) umfasst, die darauf ausgelegt ist, das Steuerungssystem (14) zu steuern.

8. Tragbares Phoroptersystem (10) nach einem der vorangegangenen Ansprüche, **dadurch gekennzeichnet, dass** das genannte Phoroptersystem (12) und das genannte Steuerungssystem (14) sich in drahtloser Kommunikation miteinander befinden.

9. Tragbares Phoroptersystem (10) nach einem der vorangegangenen Ansprüche, **dadurch gekennzeichnet, dass** das genannte Steuerungssystem (14) sich in drahtloser Kommunikation mit einem vom Steuerungssystem entfernten Server befindet.

## Revendications

1. Système de phoroptère portable (10) comprenant un système de phoroptère portable (12) associé à un système de commande (14);
ledit système de phoroptère portable (12) comprenant:
- un cadre de support (32) configuré pour être positionné à proximité d'au moins un oeil d'un sujet;
- un premier logement (26) fixé audit cadre de support (32), ledit premier logement (26) contenant un système électronique de bord;
- au moins un premier ensemble de lentilles (24) couplées audit premier logement (26) et fixées audit cadre de support (32) de sorte qu'une ouverture proximale dudit premier ensemble de lentilles (24) soit positionnée à proximité d'au moins un oeil du sujet, ledit premier ensemble de lentilles (24) comprenant:
- une lentille à puissance sphérique (54A) ayant au moins une première lentille configurée pour être actionnée électriquement et commandée par le système électronique contenu dans ledit premier logement (26), ladite première lentille étant réglable de manière continue pour corriger une puissance sphérique;
- une lentille à puissance astigmatique (52A) ayant au moins une deuxième lentille configurée pour être actionnée électriquement et commandée par le système électronique contenu dans ledit premier logement (26), ladite deuxième lentille étant réglable de manière continue pour corriger l'astigmatisme;
- une lentille aberrométrique (50A) ayant au moins une troisième lentille qui est configurée pour être actionnée et fournir un front d'onde variable commandé par le système électronique contenu dans ledit premier logement (26), ladite troisième lentille étant réglable automatiquement de manière continue pour corriger les fronts d'onde aberrants introduits au moins par la lentille à puissance sphérique (54A) et la lentille à puissance astigmatique (52A),
dans lequel ladite lentille à puissance sphérique (54A), ladite lentille à puissance astigmatique (52A) et ladite lentille aberrométrique (50A) sont alignées de façon linéaire les unes aux autres;
ledit système de commande (14) ayant une interface de phoroptère (18) configurée pour recevoir ledit système de phoroptère (12) et comprenant un émetteur de lumière (20) configuré pour transmettre de la lumière à travers le premier ensemble de lentilles (24), ledit système de phoroptère (12) étant configuré pour recevoir la lumière transmise à travers ledit premier ensemble de lentilles (24);
ledit système de commande (14) comprenant CMOS ou CCD (168) configuré pour détecter la lumière transmise à travers ledit premier ensemble de lentilles (24);
ledit système de commande (14) comprenant une ouverture d'émission de lumière (20) à travers laquelle la lumière est émise dans ledit système de phoroptère (12) positionné sur ledit système de commande (14), et une ouverture de réception de lumière (22) à travers laquelle la lumière transmise à travers ledit système de phoroptère (12) est reçue pour le traitement de la part dudit système de commande (14), pour obtenir des mesures dudit système de phoroptère (12);
dans lequel ledit système de phoroptère (12) inclut un analyseur de fronts d'onde pour effectuer des mesures aberrantes afin de corriger ladite lentille à puissance sphérique (54A) et ladite lentille à puissance astigmatique (52A) associée dans ledit système de phoroptère.

2. Ensemble de phoroptère portable (10) selon la revendication 1, **caractérisé en ce que** ledit cadre de support (32) est configuré pour être porté par un sujet sous forme de monture de lunettes.

3. Ensemble de phoroptère portable (10) selon la revendication 1, **caractérisé en ce qu'**il comprend un deuxième ensemble de lentilles (28) positionné adjacent par rapport audit premier ensemble de lentilles (24) sur ledit cadre de support (32).

4. Ensemble de phoroptère portable (10) selon la revendication 1, **caractérisé en ce que** ladite lentille à puissance sphérique (54A) a une aberration statique inférieure à 0,3 µm RMS.

5. Ensemble de phoroptère portable (10) selon la revendication 1, **caractérisé en ce que** ladite lentille à puissance astigmatique (52A) a une aberration statique inférieure à 1 µm RMS.

6. Ensemble de phoroptère portable (10) selon la revendication 1, **caractérisé en ce que** ladite lentille aberrométrique (50A) a une aberration statique inférieure à 0,8 µm RMS.

7. Ensemble de phoroptère portable (10) selon l'une des revendications précédentes, **caractérisé en ce que** ledit système de commande (14) comprend en outre un tableau de bord de contrôleur (146) configuré pour commander le système de commande (14).

8. Ensemble de phoroptère portable (10) selon l'une des revendications précédentes, **caractérisé en ce que** ledit système de phoroptère (12) et ledit système de commande (14) sont en communication sans fil l'un avec l'autre.

9. Ensemble de phoroptère portable (10) selon l'une des revendications précédentes, **caractérisé en ce que** ledit système de commande (14) est en communication sans fil avec un serveur distant loin du système de commande.
